# EUROPEAN PATENT APPLICATION

(11) **EP 2 700 943 A1**
(43) Date of publication of application: **26.02.2014**
(21) Application number: 12181708.4
(22) Date of filing: 24.08.2012
(51) Int. Cl.: G01N 33/50

(54) **In vitro method for determining pertussis toxin activity**

(71) Applicant: DE STAAT DER NEDERLANDEN VERTEGENWOORDIGD DOOR DE MINISTER VAN VOLKSGEZONDHEID, WELZIJN EN SPORT, 3720 BA Bilthoven (NL); STICHTING HOGESCHOOL UTRECHT, 3513 ES Utrecht (NL)
(72) Inventor: Akkermans, Arnoud Marinus, 3720 BA Bilthoven (NL); Pennings, Jeroen Lambertus Antonius, 3720 BA Bilthoven (NL); Vaessen, Stefan Franciscus Catharina, 3572 JE Utrecht (NL); Bruysters, Martinus Wilhelmus Petrus, 3720 BA Bilthoven (NL); Verkoeijen, Sarah, 3572 JE Utrecht (NL); Krul, Cyrille Anna Maria, 3572 JE Utrecht (NL); Vandebriel, Robert Jan, 3720 BA Bilthoven (NL)
(74) Representative: Habets, Winand

(57) **Abstract**

The invention is in the field of medicinal chemistry and provides methods and means for determining whether a composition comprises pertussis toxin activity. More specifically, the invention relates to an in vitro method for determining pertussis toxin activity in a composition, comprising the steps of providing dendritic cells (DCs), contacting the cells with the composition in a culture medium, determining the expression level of at least one gene selected from the group consisting of IL2, IFNg, XCL1, CD69, CSF2 and CXCL10, wherein the composition is likely to contain pertussis toxin activity if the expression level of at least one gene selected from the group consisting of IL2, IFNg, XCL1, CD69, CSF2 and CXCL10 is more than two times a predetermined reference value.

## Description

### Field of the invention

The invention is in the field of medicinal chemistry and provides methods and means for determining whether a composition comprises pertussis toxin activity.

### Background of the invention

Pertussis, also known as whooping cough, is a highly contagious disease caused by the bacterium Bordetella pertussis. Formerly a major cause of childhood mortality, the incidence of pertussis was greatly reduced by widespread vaccination programs in place since the 1940s. However, an increase in the number of reported cases of pertussis in the last twenty years has led public health authorities to recommend renewed and expanded vaccination efforts.

Pertussis vaccines are nowadays routinely administered to healthy infants and have proven very effective in reducing the burden of whooping cough disease. Vaccines contain, however, bacterial components as antigens and are therefore not absolutely safe.

Both whole cell and acellular pertussis vaccines are available against pertussis infection. The whole cell pertussis vaccine is composed of a suspension of formalin-inactivated B. pertussis cells. In contrast, the acellular pertussis vaccine contains purified, inactivated components of B. pertussis cells. Acellular pertussis vaccines now in use contain primarily pertussis toxin (PT) which is inactivated and filamentous hemagglutinin.

Batches of pertussis vaccine may contain residual active PT as a consequence of the production process involving B. pertussis. Because active PT is toxic to humans, regulatory authorities require safety, potency, and purity testing prior to the release of each production lot of pertussis or pertussis-containing vaccines.

With the additional notion that pertussis vaccines are given to healthy newborns, strict safety procedures are in place. In Europe these are in part registered in the European Pharmacopoeia.

Detoxified pertussis toxin (PTd), is a principal antigen in all pertussis vaccines in use today. Two tests are specifically named in the European Pharmacopeia to test for residual biologically active pertussis toxin (PT) in vaccines, i.e. the histamine sensitization test (HIST) and the Chinese Hamster Ovary (CHO) cell clustering assay. The Chinese Hamster Ovary (CHO) cell assay, measures neutralization of the biological activity of the PT by human antibodies, which is evaluated by the inhibition of toxin-mediated clustering of CHO cells. However, this test is based on cultured animal cells and in is not known how relevant these assays are for a human situation. Also, day-to-day variations in the assay performance can easily occur.

The murine histamine sensitization test (HIST) is a key safety test used to monitor residual levels of pertussis toxin in acellular pertussis vaccines. This test is performed to ensure that pertussis toxin has been effectively inactivated before release of vaccines. However, such testing may involve large numbers of mice, some of which can experience significant unrelieved pain and distress. In addition, the HIST has technical challenges requiring frequent retesting, thereby increasing vaccine testing expense.

Because the aluminum adjuvants in final vaccine formulations are toxic for CHO cells, the HIST is currently the only test that can be used for final vaccine formulations. The HIST is based on the observation that mice injected with PT are sensitized for histamine, resulting in a decrease of the lethal dose of histamine (Pittman 1975). The HIST is a lethal mouse test, although modifications have been proposed to avoid lethality (Ishida, 1979 & Ochiai 2007 & Jensen 2012). Additionally, the HIST suffers from large variations in interlaboratory test performance caused by a low uniformity in test protocols (Corbel 2004, van Straaten-van de Kapelle 1997). Together, this makes replacement of the HIST highly desirable.

Several in vitro alternatives are currently under development (Xing, 2012, Isbrucker 2010, Hoonakker 2010). These assays are based on known activities of PT, i.e. ADP-ribosylation of G proteins or the binding capacity of the toxin to specific substrate. However, not all effector mechanisms of PT are completely understood, nor are all its clinical effects (Locht 2011).

This study aims at providing alternative methods and means for determining the pertussis activity in a composition, which are able to overcome at least some of the shortcomings of the current methods.

### Summary of the invention

We have found that dendritic cells upon exposure to PT activity, show an over 50-fold increase of expression levels of interleukin 2 (IL2) and interferon gamma (IFNg) compared to control. Also four other genes showed a significant increase in their expression level upon exposure to active PT. This effect is specific since it is not found for lipopolysaccharide (LPS), another bacterial-derived vaccine component, even not when LPS was obtained from B. pertussis (LOS).

Hence, the invention relates to an in vitro method for determining pertussis toxin activity in a composition, comprising the steps of providing dendritic cells (DCs), contacting the cells with the composition in a culture medium, determining the expression level of at least one gene selected from the group consisting of IL2, IFNg, XCL1, CD69, CSF2 and CXCL10, wherein the composition is likely to contain pertussis toxin activity if the expression level of at least one gene selected from the group consisting of IL2, IFNg, XCL1, CD69, CSF2 and CXCL10 is more than two times a predetermined reference value.

### Legend to the figures

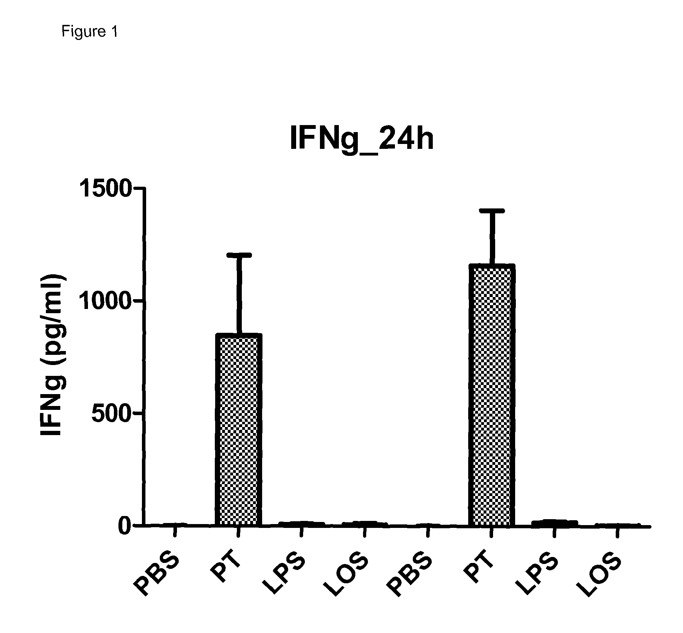

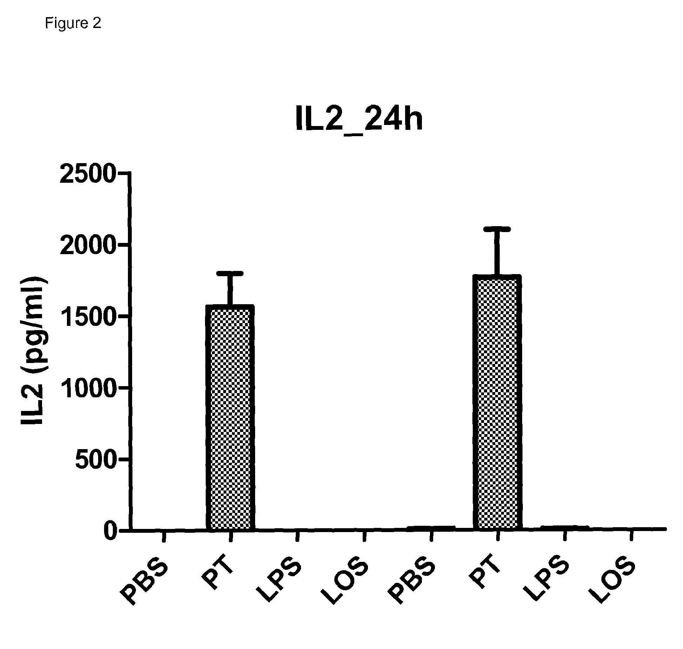

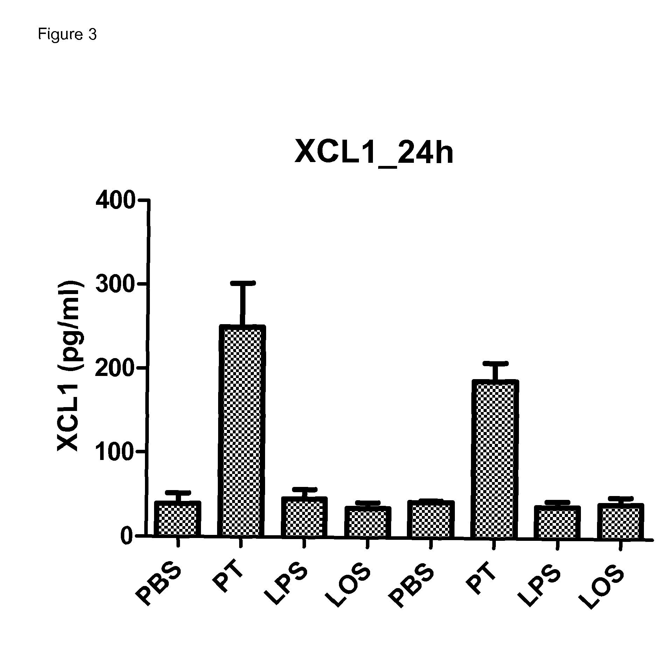

Figures 1 - 3: Protein levels of three genes which encode secreted proteins. The three genes are specifically upregulated by PT in DCs. Human monocyte derived DCs were incubated for 24 hours with control (PBS), 250 ng/ml PT, 0.1 ng/ml LPS or 0.1 ng/ml LOS. Supernatant was analyzed for interferon-gamma (IFNg), (figure 1), interleukin-2 (IL2), (figure 2) and chemokine (C motif) ligand (XCL1) (figure 3). Data from two representative donors are shown.

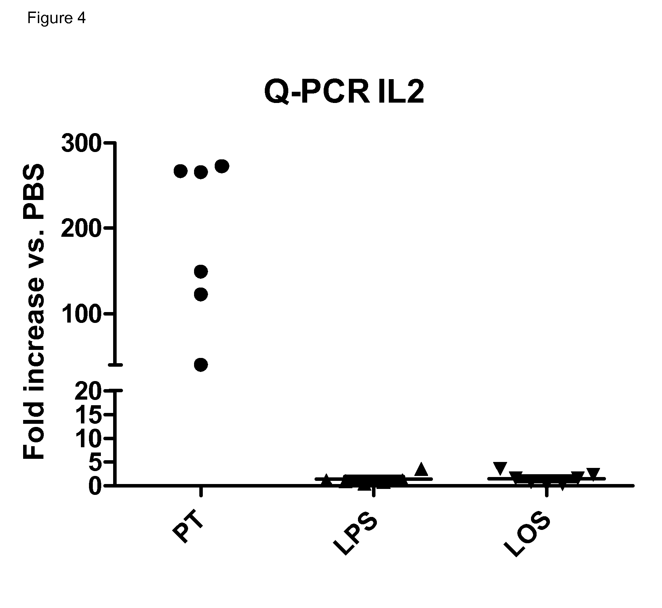

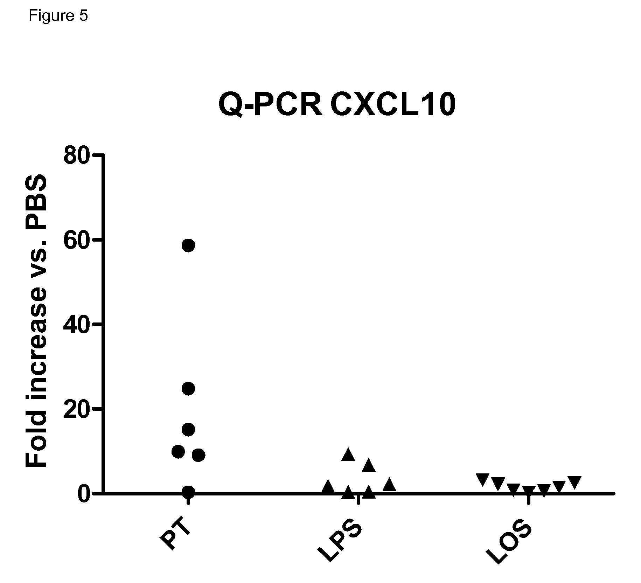

Figures 4 and 5: QPCR data obtained with dendritic cells from 6 individual donors. Expression levels of IL2 and CXCL10 confirmed the data obtained in the microarray analysis.

### Detailed description of the invention

In this study, we used dendritic cells derived from human donors in combination with gene expression analysis and ELISA. Surprisingly, we found that 6 genes were overexpressed in dendritic cells upon exposure to active PT, which was not the case in other cells. Other bacterial antigens did not affect the expression levels, therefore the finding may be used to detect PT activity.

Hence, the invention relates to an in vitro method for determining pertussis toxin activity in a composition, comprising the steps of providing dendritic cells (DCs), contacting the cells with the composition in a culture medium and determining the expression level of at least one gene selected from the group consisting of IL2, IFNg, XCL1, CD69, CSF2 and CXCL10. The composition is likely to contain pertussis toxin activity if the expression level of at least one gene selected from the group consisting of IL2, IFNg, XCL1, CD69, CSF2 and CXCL10 is more than two times a predetermined reference value.

In a preferred embodiment, more than two times is at least 3 times, such as 4, 5, 6, 7, 8, 9, 10, 11, 12, 13, 14, 15, 16, 17, 18, 19, or more than 20 times such as 25, 30, 35, 40, 45 or even more than 50 times.

Our method is animal-free and therefore attractive from the viewpoint of animal welfare. It may be performed on dendritic cells of human origin as opposed to non-human animal derived cells and it may therefore be more relevant for regulatory authorities. A method according to the invention is able to distinguish between active PT and LPS, another major vaccine constituent.

We determined the expression levels of the six genes IL2, IFNg, XCL1, CD69, CSF2 and CXCL10 in 6 different cell types (Example 2, table 2). Surprisingly, we found that all six genes were able to determine the presence of active PT in the sample when dendritic cells were used. None of the other cell types showed aberrant expression (more than 1.5 times over- or under-expression) of any of the 6 genes.

The method may be performed on all dendritic cells. Preferred are dendritic cells derived from monocytes. Particularly preferred are human dendritic cells, especially preferred are human monocyte-derived dendritic cells. In particular the genes for IL2 and IFNg were highly overexpressed in dendritic cells (Table 1).

In this study we employed dendritic cells obtained from 6 different donors. We did not observe any significant differences between the results obtained with cells from each of the individual donors, which underlines the robustness of the method.

Dendritic cells are known to the skilled person and there are several suitable ways of obtaining them. They may be derived from human tissue such as blood but the may also be derived from a cell culture. Several progenitor dendritic cell lines have been described which, upon appropriate stimulation can progress through several differentiation stages to fully mature dendritic cells. A suitable example is the MUTZ-3 cell line which is obtainable from the American Type Culture Collection ATCC and from the Deutsche Sammlung von Mikroorganismen und Zellkulturen in Leibniz, Germany..

**Table 1; gene expression data in human dendritic cells.**

| **Gene symbol** | **Gene name** | **GeneID** | **Control** | **PT** | **LPS** | **LOS** | **FDR** |
|---|---|---|---|---|---|---|---|
| IFNG | interferon, gamma | 3458 | 1 | 91,98 | 1,17 | 1,33 | 4,54E-07 |
| IL2 | interleukin 2 | 3558 | 1 | 50,29 | 1,03 | 1,11 | 2,03E-07 |
| XCL1 | chemokine (C motif) ligand 1 | 6375 | 1 | 7,46 | 1,07 | 1,09 | 5,5E-05 |
| CD69 | CD69 molecule | 969 | 1 | 8,46 | 1,24 | 1,30 | 0,000116 |
| CSF2 | colony stimulating factor 2 (granulocyte-macrophage) | 1437 | 1 | 11,13 | 1,92 | 1,25 | 0,000116 |
| CXCL10 | chemokine (C-X-C motif) ligand 10 | 3627 | 1 | 7,14 | 1,47 | 1,26 | 0,01251 |

We have found strong responses on gene expression using microarrays as well as realtime PCR. Other methods for determining the expression levels of these genes are equally suited. The skilled person is fully aware of alternative methods for determining gene expression levels.

We also were able to show the overexpression of genes in dendritic cells exposed to active PT based on the expression of excreted proteins. Figures 1 - 3 show the expression levels of IFNg, IL2 and XCI1 in the supernatant of the culture medium of the dendritic cells using ELISA. Experimental data of these methods are provided in the Examples section.

Suitable samples for determining the expression levels are therefore samples taken from the cells or the culture medium. Such a sample may either contain RNA or protein and the expression level of the genes may be determined by PCR, ELISA or microarray technology.

The amount of time required for exposure of the dendritic cells to active PT may be determined empirically and was found not to be critical. Suitable reaction times in the conditions exemplified may be about 2 hours, such as 1 or 3 hours, however this may vary considerably when different specificity or sensitivity of the assay is required.

The method may advantageously be performed with one gene selected from the group consisting of IL2, IFNg, XCL1, CD69, CSF2 and CXCL10. If one gene is used in a method according to the invention, genes IL2 and IFNg are preferred because they are the most overexpressed and therefore provide the most reliable method in terms of sensitivity and specificity.

If the reliability of the method is to be improved, the expression level of two or more genes may be determined, i.e. IL2 may be combined with any of the genes selected from the group consisting of IFNg, XCL1, CD69, CSF2 and CXCL10, IFNg may be combined with any of the genes selected from the group consisting of XCL1, CD69, CSF2 and CXCL10, XCL1 may be combined with any of the genes selected from the group consisting of CD69, CSF2 and CXCL10, CD69 may be combined with any of the genes selected from the group consisting of CSF2 and CXCL10 and CSF2 may be combined with CXCL10. In this context, the combination of II2 and IFNg is preferred

Accordingly, the performance of the method may be further improved when three genes are combined selected from the group consisting of IL2, IFNg, XCL1, CD69, CSF2 and CXCL10, i.e. genes IL2 and IFNg may be combined with at least one gene selected from the group consisting of XCL1, CD69, CSF2 and CXCL10, genes IFNg and XCL1 may be combined with at least one gene selected from the group consisting of CD69, CSF2 and CXCL10, genes XCL1 and CD69 may be combined with at least one gene selected from the group consisting of CSF2 and CXCL10 and genes CD69 and CSF2 may be combined with CXCL10.

Accordingly, the performance of the method may be further improved when four genes are combined selected from the group consisting of IL2, IFNg, XCL1, CD69, CSF2 and CXCL10, i.e. genes IL2, IFNg and XCL1 may be combined with at least one gene selected from the group consisting of CD69, CSF2 and CXCL10, genes IFNg, XCL1 and CD69 may be combined with at least one gene selected from the group consisting of CSF2 and CXCL10, genes XCL1, CD69 and CSF2 may be combined with CXCL10.

Accordingly, the performance of the method may be further improved when five genes are combined selected from the group consisting of IL2, IFNg, XCL1, CD69, CSF2 and CXCL10, i.e. the combination of genes IL2, IFNg, XCL1, CD69, and CSF2 or the combination of genes IL2, IFNg, XCL1, CD69 and CXCL10 or the combination of genes IL2, IFNg, XCL1, CSF2 and CXCL10 or the combination of genes IL2, IFNg, CD69, CSF2 and CXCL10 or the combination of genes IL2, XCL1, CD69, CSF2 and CXCL10 or the combination of genes IFNg, XCL1, CD69, CSF2 and CXCL10.

Most preferred is the combination of all 6 genes IL2, IFNg, XCL1, CD69, CSF2 and CXCL10.

The expression levels are to be compared to a reference value. Such a reference value may be empirically determined or arbitrarily chosen in order to achieve appropriate specificity and/or sensitivity of the method. A skilled person is fully aware how to choose an appropriate reference value. As an example of a suitable reference value, we used expression levels obtained with Phosphate Buffered Saline (PBS) control exposure instead of PT.

The method according to the invention is particularly suited for determining whether a vaccine is contaminated with active PT. The invention therefore particularly relates to a method as described above wherein the composition is a vaccine.

### Examples

### Example 1: Reagents

The WHO-standard pertussis toxin JNIH-5 (PT) and the European Pharmacopeia reference lipopolysaccharide E. coli 0113:H10.k (LPS) were obtained from NIBSC (Potters Bar, UK). Bordetella pertussis lipo-oligosaccharide (LOS) was a kind gift from Dr. Watanabe (Kitasato University, Tokyo, Japan.)

### Example 2: Cell culture

Six human cell types were used in this study. Human bronchial epithelial cell line BEAS-2B and human fetal lung fibroblast cell line MRC-5 were purchased from ATCC (Manassas, VA, USA). Primary human cardiac microvascular endothelial cells (HMVEC) were obtained from Lonza (Basel, Switzerland). Primary human pulmonary artery smooth mucle cells (HPASMC) were purchased from CellSystems (Troisdorf, Germany). The hybrid human cell line EA.Hy926, umbilical vein endothelial cells with epithelial cell line A549, was a kind gift from Sanquin (Amsterdam, the Netherlands).

We also cultured monocyte-derived dendritic cells (MoDCs). Briefly, peripheral blood lymphocytes were isolated from buffy coats of healthy donors by Ficoll-Paque (GE Healthcare) density gradient centrifugation and cultured in RPMI 1640 supplemented with heat-inactivated 10% FCS, 2 mM L-glutamine, 25 mM HEPES (N-2-hydroxyethylpiprazin-N'-2-ethansulfonic acid), 100 U/ml penicillin, 100 µg/ml streptomycin (all from GIBCO, Invitrogen) (hereafter defined as DC culture medium). After centrifugation cells were plated in 6-wells plates. To remove non-adherent cells, the medium was replaced after 90 minutes with DC culture medium in the presence of 100ng/ml GM-CSF and 50 ng/ml IL-4 (both Immunotools, Friesoythe, Germany). Cells were allowed to differentiate for 6 days, with cytokines replenished after 3 days, after which they were used for incubation experiments.

**Table 2; cell lines used in the study**

| **Name** | **Description** | **Origin** | **Cell line or primary culture** |
|---|---|---|---|
| BEAS2B | Broncheal epithelial cells | Human | Cell line |
| HMVEC | Cardiac microvascular endothelial cells | Human | Primary cells |
| EA.Hy926 | Hybrid of umbilical vein endothelial cells with epithelial cell line A549 | Human | Cell line |
| MRC5 | Fetal lung fibroblast cells | Human | Cell line |
| HPASMC | Pulmonary artery smooth muscle cells | Human | Primary cells |
| MoDCs | Monocyte-derived dendritic cells | Human | Primary cells |

### Example 3: Incubations for microarray experiments

Incubations were performed in 6 wells plates. Cells were incubated for 2 hours with 1 ml of 250 ng/ml (250 IU/ml) PT, 1 EU/ml (=0.1 ng/ml) LPS, 0.1 ng/ml LOS or with PBS as a negative control. Each experimental group consisted of four replicates of which the cells were directly collected in RNA protect (Qiagen, Venlo, The Netherlands) to stabilize RNA. Total RNA was purified using the RNeasy plus mini kit (Qiagen) according to the manufacturer's instructions. The quantity of RNA in each sample was measured using a NanoDrop Spectrophotometer (NanoDrop technologies, Wilmington, DE), and RNA integrity was assessed on the 2100 Bioanalyzer (Agilent Technologies, Amstelveen, The Netherlands) using the RNA 6000 Nano Chip Kit (Agilent Technologies).

### Example 4: Microarray experiments

RNA was further processed for hybridization to Affymetrix HT HG-U133+ PM Array Plates. RNA amplification, labeling and genechip hybridization, washing and scanning were carried out according to Affymetrix protocols. For the cell line comparison, 1-2 biological replicates were used for each group. For the MoDC analysis, 4 biological replicates were used per group. Quality control and normalization were performed using the pipeline at the www.arrayanalysis.org website (Maastricht University, the Netherlands). Normalization was done using the Robust Multichip Average (RMA) algorithm (Irizarry, 2003) and the MBNI custom CDF (Dai, 2005) version #14 for this chip. All slides passed the various quality control steps. Normalization output consisted of data for 18909 probe sets each corresponding to unique Entrez GeneIDs.

### Example 5: Data analysis microarray experiments

Gene expression data between (two or more) experimental groups were compared using ANOVA. Obtained p-values were corrected for multiple testing by calculating the false discovery rate (FDR) according to Benjamini and Hochberg (Benjamini 1995). Probe sets with a False Discovery Rate (FDR) < 10% and a Fold Ratio > 1.5 compared to the matched control groups were considered significantly differentially expressed.

### Example 6: Microarray verification on RNA level with quantitative PCR

To confirm the microarray data, mRNA of MODCs used for the microarray analysis and of 2 additional incubations performed similarly as for microarray analysis was converted to cDNA using the high capacity cDNA Reverse Transcription Kit (Applied Biosystems) according to the manufacturer's protocol. Relative Expression of IL2 and CXCL10 was quantified after normalization using reference genes HPRT1 and POLR2A using specific Taqman gene expression assays and Taqman Fast universal PCR master mix (both Applied Biosystems), according to supplied protocols on a 7500 Fast Realtime PCR systems (Applied Biosystems).

The following assays were used in the gene analysis:
- HPRT1: assay: Hs02800695_m1
- POLR2A: assay Hs00172187_m1
- IL2:: Hs00174114_m1
- CXCL10:: Hs01124251_g1

Genes HPRT1 en POLR2A are housekeeping genes. The results are shown in figures 4 and 5 and confirm the data obtained in the microarrays.

### Example 7: Microarray verification on protein level using ELISA

To verify the microarray data on the protein level, PBMCs were isolated as described above and differentiated as described above in 24-well culture plates. MoDCs were incubated with 250 ng/ml (250 IU/ml) PT, 1 EU/ml (=0.1 ng/ml) LPS, 0.1 ng/ml LOS or with PBS as a negative control for 24 hours after which supernatant was stored at -80°C for further analysis. Protein levels of interleukin-2 (IL-2), interferon-gamma (IFN-Y), Chemokine (C motif) ligand (XCL1) and C-X-C motif chemokine 10 (CXCL10) were determined in supernatant using specific commercially available ELISAs (IL-2 and IFN-Y: eBiosciences, San Diego, CA and XCL1 and CXCL10: R&D Systems, Minneapolis, MN).

### Example 8: Statistical analysis of qPCR and ELISA data

Results are presented as means and standard deviation. Comparisons between different treatmens were done using a one-way analysis of variance (ANOVA) with a Bonferroni post-hoc test. A p-value <0.05 was considered statistically significant.

### Example 9: Effect of pertussis toxin on mRNA expression levels in selected human cell types

We examined the effect of PT on gene expression in six different human cell types that are implicated in the effects of PT in humans (table 2). Cells were incubated for 2 hours with either 250 ng/ml PT or with a PBS control, after which microarray analysis was performed. Cell line expression data were corrected for their corresponding control group. Statistical analysis found 65 genes differentially expressed in dendritic cells DCs), in particular in monocyte-derived dendritic cells. We found no significantly regulated genes for any of the individual other cell lines.

### Example 10: Pertussis toxin-sensitive genes in DCs

To confirm the data obtained with DCs in the first experiment, we repeated the experiment with DCs from four different donors. We included in this analysis incubations with bacterial lipopolysaccharides (Eur. Pharmacopoeia, LPS) and with LPS isolated from B. pertussis (LOS).

### Example 11 Gene expression analysis of 6 genes

Genes IFNG, IL2, XCL1, CD69, CSF2, CXCL10 were found to be significantly upregulated upon exposure of dendritic cells to PT. (Table 1). Quantitative RT-PCR for IL2 and CXCL10 confirmed the microarray results.

In addition to the gene expression data, we also measured protein levels of the genes that encode secreted proteins in the medium of monocyte-derived human DCs after a 24 hour incubation with PBS, PT, LPS and LOS. Protein levels of interleukin-2, interferon-gamma and Chemokine (C motif) ligand (XCL1) reflect the observed effects on gene expression.

### References

1. Nishida M, Suda R, Nagamatsu Y, Tanabe S, Onohara N, Nakaya M, Kanaho Y, Shibata T, Uchida K, Sumimoto H, Sato Y, Kurose H. Pertussis toxin up-regulates angiotensin type 1 receptors through Toll-like receptor 4-mediated Rac activation. J Biol Chem. 2010 May 14;285(20):15268-77.
2. Hamaguchi I, Imai J, Momose H, Kawamura M, Mizukami T, Kato H, Naito S, Maeyama J, Masumi A, Kuramitsu M, Takizawa K, Mochizuki M, Ochiai M, Yamamoto A, Horiuchi Y, Nomura N, Watanabe S, Yamaguchi K. Two vaccine toxicity-related genes Agp and Hpx could prove useful for pertussis vaccine safety control. Vaccine. 2007 Apr 30;25(17):3355-64.
3. Hamaguchi I, Imai J, Momose H, Kawamura M, Mizukami T, Naito S, Maeyama J, Masumi A, Kuramitsu M, Takizawa K, Kato H, Mizutani T, Horiuchi Y, Nomura N, Watanabe S, Yamaguchi K. Application of quantitative gene expression analysis for pertussis vaccine safety control. Vaccine. 2008 Aug 26;26(36):4686-96.
4. Benjamini Y, Hochberg Y (1995) Controlling the false disovery rate: a practical and powerful approach to multiple testing. J R Stat Soc B 57: 289-300.
5. Pittman, M. 1975. Determination of the histamine sensitizing unitage of pertussis vaccine. J. Biol. Stand. 3:185-191.
6. Ishida S, Kurokawa M, Asakawa S, Iwasa S. A sensitive assay method for the histamine-sensitizing factor using change in rectal temperature of mice after histamine challenge as a response. J Biol Stand. 1979 Jan;7(1):21-9.
7. Ochiai M, Yamamoto A, Kataoka M, Toyoizumi H, Arakawa Y, Horiuchi Y. Highly sensitive histamine-sensitization test for residual activity of pertussis toxin in acellular pertussis vaccine. Biologicals. 2007 Oct;35(4):259-64.
8. Jensen SE, Illigen KE, Badsberg JH, Hasløv KR. Specificity and detection limit of a dermal temperature histamine sensitization test for absence of residual pertussis toxin in vaccines. Biologicals. 2012 Jan;40(1):36-40.
9. Corbel MJ, Xing DK. Toxicity and potency evaluation of pertussis vaccines. Expert Rev Vaccines. 2004 Feb;3(1):89-1 01.
10. van Straaten-van de Kappelle I, van der Gun JW, Marsman FR, Hendriksen CF, van de Donk HJ. Collaborative study on test systems to assess toxicity of whole cell pertussis vaccine. Biologicals. 1997 Mar;25(1):41-57.
11. Xing D, Yuen CT, Asokanathan C, Rigsby P, Horiuchi Y. (2012) valuation of an in vitro assay system as a potential alternative to current histamine sensitization test for acellular pertussis vaccines. Biologicals. , in press. http://dx.doi.org/10.1016/j.biologicals.2012.07.013
12. Isbrucker RA, Bliu A, Prior F. (2010) Modified binding assay for improved sensitivity and specificity in the detection of residual pertussis toxin in vaccine preparations. Vaccine. ;28(15):2687-92.
13. Hoonakker ME, Ruiterkamp N, Hendriksen CF. (2010) The cAMP assay: a functional in vitro alternative to the in vivo Histamine Sensitization test. Vaccine. ;28(5):1347-52.
14. Locht C, Coutte L, Mielcarek N. (2011) The ins and outs of pertussis toxin. FEBS J. ;278(23):4668-82.
15. Irizarry RA, Bolstad BM, Collin F, Cope LM, Hobbs B, et al. (2003) Summaries of Affymetrix GeneChip probe level data. Nucleic Acids Res 31: e15.
16. Dai M, Wang P, Boyd AD, Kostov G, Athey B, et al. (2005) Evolving gene/transcript definitions significantly alter the interpretation of GeneChip data. Nucleic Acids Res 33: e175.

## Claims

1. In vitro method for determining pertussis toxin activity in a composition, comprising the steps of:
a. Providing dendritic cells (DCs),
b. Contacting the cells with the composition in a culture medium,
c. Determining the expression level of at least one gene selected from the group consisting of IL2, IFNg, XCL1, CD69, CSF2 and CXCL10.
wherein said composition is likely to contain pertussis toxin activity if the expression level of at least one gene selected from the group consisting of IL2, IFNg, XCL1, CD69, CSF2 and CXCL10 is more than two times a predetermined reference value.

2. Method according to claim 1 wherein the dendritic cells are monocyte-derived dendritic cells.

3. Method according to claims 1 or 2 wherein the dendritic cells are human cells.

4. Method according to claims 1 - 3 wherein the expression of the genes is measured in a sample taken from the cells or a culture medium.

5. Method according to claim 4 wherein the sample contains RNA and/or protein

6. Method according to claims 1 -5 wherein the expression level of the genes is determined by PCR, ELISA or microarray technology.

7. Method according to claims 1 - 6 wherein the cells are contacted with the composition for at least 2 hours.

8. Method according to claims 1 7 wherein the at least one gene is at least two genes, such as at least three genes, such as at least four genes, such as at least five genes most preferred six genes.

9. Method according to claims 1 - 8 wherein the predetermined reference value is a value obtained with a Phosphate Buffered Saline (PBS) control exposure.

10. Method according to claims 1 - 9 wherein the composition is a vaccine.

11. Method according to claims 1 - 10 wherein the at least one gene is selected from the group consisting of IFNg and IL2.

12. Method according to claim 11 wherein additionally the expression level of at least one gene is determined selected from the group consisting of XCL1, CD69, CSF2 and CXCL10.

13. Method according to claim 12 wherein the at least one gene is at least two genes, such as at least three genes, such as four genes.

14. Method according to claims 1 - 13 wherein the more than two times the reference value is more than 3, 4, 5, 6, 7, 8, 9, or 10 times the reference value.

15. Method according to claims 1 - 14 wherein the reference value is the reference value of the respective gene obtained when the dendritic cell is exposed to a PBS control.
